# FASCICULE DE BREVET EUROPEEN

(11) **EP 0 542 625 B1**
(45) Date de publication et mention de la délivrance du brevet: **28.06.1995**
(21) Numéro de dépôt: 92403049.7
(22) Date de dépôt: 12.11.1992
(51) Int. Cl.: B01J 29/04, B01J 29/40, C07C 2/00

(54) **Catalyseur de type aluminosilicate et son utilisation en aromatisation d'hydrocarbures comportant 2 à 12 atomes de carbone**
Aluminosilikat Katalysator und seine Verwendung zum Aromatisieren von 2 bis 12 Kohlenstoffatome haltende Kohlenwasserstoffen
Aluminosilicate type catalyst and its use for the aromatizing of hydrocarbons containing 2 to 12 carbon atoms

(30) Priorité: 15.11.1991 FR 9114205; 15.11.1991 FR 9114207
(43) Date de publication de la demande: 19.05.1993
(73) Titulaire: INSTITUT FRANCAIS DU PETROLE, 92502 Rueil-Malmaison (FR)
(72) Inventeur: Alario, Fabio, F-92200 Neuilly sur Seine (FR); Deves, Jean-Marie, F-78400 Chatou (FR)

(56) Documents cités:
- EP-A- 0 018 498
- EP-A- 0 244 162
- EP-A- 0 351 311
- EP-A- 0 458 674
- EP-A- 0 469 951
- US-A- 4 806 701
- US-A- 4 808 763

## Description

La présente invention concerne un catalyseur, dit catalyseur composite, qui contient :
- une zéolithe de structure MFI sous forme hydrogène, contenant dans sa charpente le silicium et au moins un élément choisi dans le groupe formé par l'aluminium et le gallium,
- une matrice, laquelle renferme :
- au moins un métal noble de la famille du platine, au moins un métal additionnel choisi dans le groupe constitué par l'étain le, germanium et le plomb.
- au moins un halogène choisi dans le groupe constitué par le fluor, le chlore, le brome et l'iode,

L'invention concerne également la préparation du catalyseur et son utilisation dans les réactions d'aromatisation des hydrocarbures comportant de 2 à 12 atomes de carbone par molécule et plus particulièrement de 5 à 12 atomes de carbone par molécule, ou 2 à 4 atomes de carbone par molécule selon les types de charges utilisées.

Les catalyseurs à base de zéolithes dopées au platine, sont connus pour être actifs et sélectifs en aromatisation du propane et du butane. Classiquement, les hydrocarbures à plus de 6 atomes de carbone par molécule sont transformés en aromatiques par reformage catalytique en utilisant des catalyseurs du type alumine acide contenant du platine, métal auquel on peut ajouter par exemple de l'étain ou du rhénium. Ces catalyseurs de reformage sont néanmoins très peu performants pour l'aromatisation des hydrocarbures contenant moins de 6 atomes de carbone par molécule. Il y a donc un grand intérêt pratique à trouver des catalyseurs performants pour l'aromatisation des coupes riches en hydrocarbures du type C₅ - C₁₂.

La réaction d'aromatisation des hydrocarbures contenant moins de 9 atomes de carbone par molécule en présence de zéolithes a déjà fait l'objet de brevets et de publications. Plusieurs systèmes catalytiques à base de zéolithe MFI sont revendiqués, ces systèmes pouvant se distinguer par les ajouts qu'ils contiennent. Schématiquement, on peut distinguer :
(i) les systèmes dopés au gallium (US-A-4175057), et,
(ii) les systèmes dopés au zinc (US-A-4288645).

Ces systèmes souffrent tous d'un défaut important, à savoir une sélectivité élevée en méthane. Pour améliorer les performances de ces systèmes catalytiques plusieurs solutions ont été proposées dont l'ajout de platine (Z. Jin, Y. Makino, A. Miyamoto, T. Inui, Chem. Express, 2, p.515, 1987). L'utilisation d'une zéolithe MFI non acide dopée avec divers éléments métalliques a également été revendiquée (Y Chen et al., WO 8904818).

Récemment (EP-A-0530069 de la demanderesse qui appartient à l'état de la technique visé à l'article 54 (3) CBE), il a été découvert que l'utilisation de catalyseurs composites contenant une zéolithe MFI d'une part, et d'autre part un support ou une matrice généralement amorphe sur laquelle est déposé un métal noble de la famille du platine et au moins un métal additionnel comme l'étain, le plomb ou l'indium, conduit à des performances catalytiques dans les réactions d'aromatisation des paraffines de 5 à 9 atomes de carbone nettement améliorées par rapport aux systèmes de l'art antérieur.

L'utilisation de tels catalyseurs permet en particulier de limiter les réactions qui conduisent à la formation de méthane, produit non désiré.

Les travaux de recherche effectuées par la demanderesse l'ont conduite à découvrir que, de façon surprenante, l'utilisation d'un catalyseur composite contenant une zéolithe MFI sous forme hydrogène, éventuellement du gallium et/ou du zinc sous forme oxyde et une matrice sur laquelle est déposé au moins un métal noble de la famille du platine (notamment palladium, platine, nickel, iridium, rhodium), au moins un métal additionnel choisi dans le groupe constitué par l'étain, le germanium, et le plomb, ladite matrice contenant aussi au moins un halogène et éventuellement au moins un métal alcalin ou un alcalino-terreux (de préférence le lithium ou le potassium), conduit à des performances catalytiques dans les réactions d'aromatisation des paraffines contenant de 5 à 12 atomes de carbone par molécule nettement améliorées par rapport aux catalyseurs de l'art antérieur.

La zéolithe MFI contenue dans le catalyseur de la présente invention peut être préparée par toutes les techniques décrites dans l'art antérieur. Ainsi la synthèse de ladite zéolithe peut être réalisée en milieu classique OH- en présence ou en absence d'agent organique et/ou d'alcool. Le document "Synthesis of high silica zeolites, P. Jacobs and J. Martens, Studies in Surface Science and Catalysis, Vol. 33, 1987" décrit la synthèse classique de la zéolithe MFI. La zéolithe MFI utilisée dans la présente invention peut également avoir été synthétisée dans des milieux moins classiques comme par exemple le milieu fluorure en présence (brevet EP-A-172068) ou en absence (demande de brevet français 90/16529) de composé organique. La zéolithe utilisée dans la présente invention contient, dans sa charpente cristallisée, du silicium et au moins un élément choisi dans le groupe formé par l'aluminium et le gallium.

Après l'étape de synthèse, la zéolithe MFI est transformée en une forme hydrogène, notée H-MFI, par élimination pratiquement totale des composés organiques et/ou des cations alcalins ou alcalino-terreux qu'elle contient éventuellement après synthèse. Toutes les techniques décrites dans l'art antérieur peuvent être utilisées pour le passage à la forme hydrogène, comme par exemple les échanges ioniques suivis ou non de calcination ou les traitements chimiques divers.

Toutes les zéolithes synthétisées dans l'un des systèmes suivants : Si-Al, Si-Al-Ga, Si-Ga, conviennent pour la présente invention. Cependant, leur rapport Si/T où T représente Al et/ou Ga, est généralement supérieur à 7, de préférence supérieur à 10 et de manière encore plus préférée compris entre 13 et 500.

La zéolithe H-MFI, utilisée dans la présente invention, peut être éventuellement soit éventuellement soumise telle quelle à un dépôt de gallium et/ou de zinc, soit mélangée avec les autres constituants du catalyseur, le gallium et/ou le zinc pouvant alors éventuellement être introduits ultérieurement dans ledit mélange (sur la zéolithe et/ou la matrice).

De nombreuses techniques de dépôt de gallium et/ou de zinc peuvent être utilisées dans la présente invention, parmi lesquelles on peut citer les échanges ioniques grâce à l'utilisation de sels en solution aqueuse, ou les imprégnations par des solutions desdits sels.

La teneur cumulée en ces deux éléments dopeurs éventuellement déposés sur le catalyseur composite est comprise entre 0,01 et 10 % en poids, de préférence entre 0,03 et 4 % en poids.

La matrice comprend au moins un oxyde réfractaire, et en particulier au moins un oxyde d'un métal choisi dans le groupe constitué par le magnésium, l'aluminium, le titane, le zirconium, le thorium, le silicium et le bore. De plus, elle peut aussi comprendre du charbon.

La matrice préférée est l'alumine, dont la surface spécifique peut être avantageusement comprise entre 10 et 600 m2/g et de préférence entre 150 et 400 m²/g.

Le catalyseur composite de la présente invention peut être préparé suivant le voie dont le principe est donné ci-après, la réalisation pratique étant connue de l'homme de l'art.

On dépose préalablement les métaux et l'halogène sur la matrice d'une part, et éventuellement le gallium et/ou le zinc sur la zéolithe H-MFI d'autre part. L'ordre d'introduction des éléments est indifférent. Puis on procède au mélange de la zéolithe H-MFI contenant éventuellement du gallium et/ou du zinc avec la matrice contenant les métaux et l'halogène et à leur mise en forme, la mise en forme étant obtenue dans les mêmes conditions que précédemment.

La méthode préférée de préparation consiste, à déposer les métaux et à introduire l'halogène sur la matrice, puis ensuite à introduire la zéolithe (éventuellement chargée en gallium et/ou en zinc) dans la matrice chargée en métaux par mise en forme des deux poudres. La mise en forme est de préférence effectuée après un broyage micronique, qui peut être réalisé en utilisant la technique du broyage humide.

Le catalyseur composite contient entre 1 et 99 % en poids de zéolithe, le complément à 100 % étant constitué par la matrice, les métaux et l'halogène. La proportion respective de zéolithe et de matrice varie dans une large gamme, car elle dépend d'une part du rapport Si/T de la zéolithe, où T est Al et/ou Ga, et d'autre part de la teneur en métaux et en halogène de la matrice dans le cas de la méthode préférée de préparation.

La matrice contenant les métaux et l'halogène, dans le cas de la méthode préférée de préparation, est généralement préparée suivant les procédures décrites dans EP-A-0530069 de la demanderesse, dont une partie est reproduite dans ce qui suit.

On utilise pour l'imprégnation des métaux, soit une solution commune des métaux que l'on désire introduire, soit des solutions distinctes pour le métal noble de la famille du platine et pour le métal additionnel et éventuellement l'élément choisi dans le groupe constitué par les métaux alcalins et les alcalino-terreux. Quand on utilise plusieurs solutions, on peut procéder à des séchages et/ou à des calcinations intermédiaires. On termine habituellement par une calcination, par exemple entre 500 et 1000 °C, de préférence en présence d'oxygène moléculaire, par exemple en effectuant un balayage d'air.

Le métal noble de la famille du platine peut être incorporé dans la matrice par imprégnation de ladite matrice à l'aide d'une solution, aqueuse ou non, contenant un sel ou un composé du métal noble. Le platine est généralement introduit dans la matrice sous forme d'acide chloroplatinique, mais pour tout métal noble peuvent être également utilisés des composés ammoniaqués ou des composés tels que par exemple le chloroplatinate d'ammonium, le dichlorure de platine dicarbonyle, l'acide hexahydroxyplatinique, le chlorure de palladium, le nitrate de palladium.

Le métal additionnel choisi dans le groupe constitué par l'étain, et le germanium, le plomb, peut être introduit par l'intermédiaire de composés tels que par exemple les chlorures, les bromures et les nitrates d'étain, les halogénures, le nitrate, l'acétate et le carbonate de plomb, le chlorure et l'oxalate de germanium.

L'halogène peut être introduit à partir d'au moins un des halogénures des métaux de la famille du platine ou d'au moins un des halogénures des métaux additionnels, dans le cas où ces métaux sont introduits à partir d'halogénures. Une méthode complémentaire peut consister en une imprégnation de la matrice par une solution aqueuse contenant un acide ou un sel halogéné. Par exemple, le chlore peut être déposé en utilisant une solution d'acide chlorhydrique. Une autre méthode peut consister en une calcination à une température généralement comprise entre 400 et 900° C, en présence d'un composé organique contenant l'halogène, comme par exemple CCl₄, CH₂Cl₂, CH₃Cl,...).

L'élément optionnel choisi dans le groupe constitué par les alcalins et les alcalino-terreux, de préférence le lithium ou le potassium, peut être introduit par l'intermédiaire de composés tels que par exemple l'halogénure, le nitrate, le carbonate, le cyanure et l'oxalate dudit élément.

Un procédé de préparation, que nous détaillons ci-après, comprend par exemple les étapes suivantes :
a) introduction sur la matrice d'au moins un élément choisi dans le groupe constitué par le fluor, le chlore, le brome et l'iode,
a') Introduction éventuelle sur la matrice d'au moins un élément choisi dans le groupe constitué par les alcalins et les alcalino-terreux.
b) Calcination du produit obtenu à l'étape a) ou a').
c) Introduction sur la matrice d'au moins un métal noble de la famille du platine.
d) Calcination du produit obtenu à l'étape c).
e) Introduction sur le produit obtenu à l'étape d) d'au moins un métal additionnel M.

Si on n'utilise pas de métal alcalin ou alcalino-terreux, on effectue uniquement les étapes a), c), d) et e) dudit procédé de préparation.

L'emploi dans la présente invention d'au moins un métal noble de la famille du platine peut à titre d'exemple se faire grâce à l'utilisation de composés ammoniaqués.

Dans le cas du platine, on peut citer par exemple les sels de platine IV hexamines de formule Pt(NH₃)₆X₄; les sels de platine IV halogénopentamines de formule (PtX(NH₃ )₅)X₃; les sels de platine N tétrahalogénodiamines de formule PtX₄(NH₃)₂ ; les complexes de platine avec les halogènes-polycétones et les composés halogénés de formule H(Pt(aca)₂X) ; X étant un halogène choisi dans le groupe formé par le chlore, le fluor, le brome et l'iode, et de préférence X étant le chlore, et aca représentant le reste de formule C₅H₇O₂ dérivé de l'acétylacétone.

L'introduction du métal noble de la famille du platine est de préférence effectuée par imprégnation à l'aide d'une solution aqueuse ou organique de l'un des composés organométalliques cités ci-dessus. Parmi les solvants organiques utilisables, on peut citer les hydrocarbures paraffiniques, naphténiques ou aromatiques, et les composés organiques halogénés ayant par exemple de 1 à 12 atomes de carbone par molécule. On peut citer par exemple le n-heptane, le méthylcyclohexane, le toluène et le chloroforme. On peut aussi utiliser les mélanges de solvants.

Après introduction du métal noble de la famille du platine, le produit obtenu est éventuellement séché puis il est calciné de préférence à une température comprise entre 400 et 1000° C.

Après cette calcination, on procède à l'introduction d'au moins un métal additionnel, précédée éventuellement d'une réduction à l'hydrogène à haute température, par exemple entre 300 et 500° C. Le métal additionnel appelé M ci-dessous peut être introduit sous la forme d'au moins un composé organique choisi dans le groupe constitué par les complexes dudit métal, en particulier les complexes polycétoniques du métal M et les hydrocarbylmétaux tels que les alkyles, les cycloalkyles, les aryles, les alkylaryles et les arylalkyles métaux.

L'introduction du métal M est avantageusement effectuée à l'aide d'une solution du composé organométallique dudit métal dans un solvant organique. On peut également employer des composés organohalogénés du métal M. Comme composés du métal M, on peut citer en particulier le tétrabutylétain dans le cas où M est l'étain, le tétraéthylplomb dans le cas où M est le plomb,

Le solvant d'imprégnation est choisi dans le groupe constitué par les hydrocarbures paraffiniques, naphténiques ou aromatiques contenant de 6 à 12 atomes de carbone par molécule et les composés organiques halogénés contenant de 1 à 12 atomes de carbone par molécule. On peut citer par exemple le n-heptane, le méthylcyclohexane et le chloroforme. On peut aussi utiliser des mélanges des solvants définis ci-dessus.

Dans le cas où l'on n'utilise pas le procédé de préparation tel que décrit ci-dessus, on peut aussi envisager d'introduire au moins un métal additionnel M avant l'introduction d'au moins un métal noble de la famille de platine. Si le métal M est introduit avant le métal noble, le composé du métal M utilisé est généralement choisi dans le groupe constitué par l'halogénure, le nitrate, l'acétate, le tartrate, le carbonate et l'oxalate du métal M. L'introduction est alors avantageusement effectuée en solution aqueuse. Dans ce cas, avant de procéder à l'introduction d'au moins un métal noble, on procède à une calcination sous air à une température comprise entre 400 et 1000° C.

Le catalyseur composite contient :
1) en poids par rapport à la matrice:
   - de 0,01 à 2 % et de préférence de 0,1 à 0,5 % d'au moins un métal noble de la famille du platine,
   - au moins un métal additionnel dont la concentration est de 0,005 à 2 %, de préférence 0,01 à 0,5 %, d'étain dans le cas où le métal additionnel est l'étain, de 0,005 à 0,7 %, de préférence 0,01 à 0,6 %, d'au moins un métal additionnel autre que l'étain (choisi dans le groupe constitué par le germanium, le plomb) ; dans le cas où le catalyseur contient deux métaux additionnels, la teneur globale en métaux additionnels est comprise entre 0,02 et 1,20 %, de préférence entre 0,02 et 1,0 %, et de manière encore plus préférée entre 0,03 et 0,80 %.
   - de 0,1 à 15 %, de préférence de 0,2 à 10 %, d'un élément choisi dans le groupe constitué par le fluor, le chlore, le brome et l'iode, de préférence le chlore,
   - éventuellement de 0,01 à 4 %, de préférence de 0,1 à 0,6 % d'au moins un métal choisi dans le groupe constitué par les alcalins et les alcalino-terreux, de préférence choisi dans le groupe constitué par le lithium et le potassium.
2) entre 1 et 99 % en poids de zéolithe MFI forme hydrogène contenant dans sa charpente du silicium et au moins un élément choisi parmi l'aluminium et le gallium,
3) et éventuellement en poids par rapport à la zéolithe :
   - entre 0,01 et 10% en poids, de préférence entre 0,03 et 4%, d'un élément dopeur choisi dans le groupe constitué par le gallium et le zinc, de préférence le gallium, cet élément étant introduit dans la zéolithe et/ou la matrice.

A l'issue de sa préparation, le catalyseur mis en forme contient une zéolithe H-MFI, des métaux, au moins un halogène et une matrice, et on procède à une calcination sous air à une température comprise entre 450 et 1000° C. Le catalyseur ainsi calciné peut avantageusement subir un traitement d'activation sous hydrogène à haute température, par exemple comprise entre 300 et 500° C. La procédure de traitement sous hydrogène consiste par exemple en une montée lente de la température sous courant d'hydrogène jusqu'à la température maximale de réduction, comprise généralement entre 300 et 500° C et de préférence entre 350 et 450° C, suivie d'un maintien à cette température pour une durée comprise en général entre 1 et 6 heures.

Le catalyseur de la présente invention décrit précédemment est mis en oeuvre pour l'aromatisation des alcanes contenant de 2 à 12, par exemple 2 à 4 ou 5 à 12 atomes de carbone par molécule, en présence ou non d'oléfines. Cette réaction revêt un intérêt particulier car elle peut permettre, par exemple, d'une part de valoriser des fractions légères provenant d'opérations de raffinage en les transformant en des produits à plus haute valeur ajoutée (benzène, toluène et xylènes), d'autre part de transformer des charges paraffiniques pouvant contenir des oléfines en bases pour carburant à haut indice d'octane, tout en contribuant dans les deux cas à la production de quantités importantes d'hydrogène indispensable pour les procédés d'hydrotraitement par exemple.

La charge qui contient des composés contenant de 5 à 12 atomes de carbone par molécule est mise en contact avec le catalyseur de la présente invention à une température comprise entre 400 et 700° C.

Les exemples qui suivent précisent l'invention sans toutefois en limiter la portée.

### Exemple 1 : préparation de l'alumine renfermant du platine, de l'étain et du chlore (catalyseur A).

L'alumine utilisée a une surface spécifique de 220 m²/g et un volume poreux de 0,52 cm³/g.

A 100 g de support d'alumine on ajoute 500 cm³ d'une solution aqueuse d'acide chlorhydrique. On laisse en contact 3 heures, on essore, on sèche 1 heure à 100-120° C.

Sur le produit séché contenant le chlore, on procède alors à l'imprégnation du platine, en ajoutant au solide 150 cm³ d'une solution aqueuse d'acide hexachloroplatinique. La concentration en platine de cette solution est égale à 2.7 g/l. On laisse 6 heures en contact, on sèche 1 heure à 100-120° C puis on calcine 2 heures à 530° C .

Sur le produit calciné contenant le chlore et le platine, on procède à l'imprégnation de l'étain : une solution organique de tétrabutylétain est mise en contact avec le support d'alumine à raison de 100 cm3 de solution pour 100 g de support pendant 6 heures. Le solide obtenu est alors essoré et séché 1 heure à 100-120° C puis réduit sous courant d'hydrogène sec pendant 2h à 450° C.

L'alumine contient alors, en poids, 0,40 % de platine, 0,3 % d'étain et 1,0 % de chlore.

### Exemple 2 : zéolithe MFI forme hydrogène (catalyseur B)

On utilise une zéolithe H-MFI forme hydrogène qui est obtenue de la forme NaMFI issue de la synthèse de la façon suivante :
- traitement au nitrate d'ammonium
- calcination à 550° C sous air.

Les caractéristiques du solide obtenu sont : un rapport Si/Al égal à 29 et une teneur en sodium égale à 0,014 % en poids. Son volume poreux mesuré par adsorption d'azote à 77K est de 0,192 cm³/g. Le volume de maille du réseau cristallin est de 5339 Angstroëm cube.

### Exemple 3 : préparation des mélanges (catalyseurs D et E)

### Préparation de l'alumine renfermant du platine et de l'étain (catalyseur C).

L'alumine utilisée est identique à celle utilisée dans l'exemple 1.

Sur 100 g de cette alumine on procède à l'imprégnation du platine, en ajoutant au solide 150 cm3 d'une solution aqueuse d'acide hexachloroplatinique. La concentration en platine de cette solution est égale à 2,7 g/l. On laisse 6 heures en contact, on sèche 1 heure à 100-120° C puis on calcine 2 heures à 530° C .

Sur le produit calciné contenant le platine, on procède à l'imprégnation de l'étain : une solution organique de tétrabutylétain est mise en contact avec le support d'alumine à raison de 100 cm³ de solution peur 100 g de support pendant 6 heures. Le solide obtenu est alors essoré et séché 1 heure à 100-120° C puis réduit sous courant d'hydrogène sec pendant 2 heures à 450° C.

L'alumine ainsi préparée (catalyseur C) contient alors, en poids, 0,40 % de platine, et 0,3 % d'étain.

### Préparation des mélanges (catalyseurs D et E)

Les catalyseurs D et E sont préparés par mélange à partir des catalyseurs A, B et C. Ces mélanges sont réalisés dans les proportions massiques suivantes :
- catalyseur D = 60 g catalyseur A + 40 g catalyseur B
- catalyseur E = 60 g catalyseur C + 40 g catalyseur B

Chacun des constituants de ces mélanges est soumis au préalable à un broyage submicronique. Après mélange, les catalyseurs D et E sont mis en forme par pastillage.

### Exemple 4 : performances des catalyseurs sur charge contenant 5 et 6 atomes de carbone par molécule

On se propose de transformer une charge constituée d'un mélange d'hydrocarbures contenant de 5 à 6 atomes de carbone par molécule. On opère pour cela en présence d'un des quatre catalyseurs A, B, D et E dont la préparation a été décrite précédemment.

Ces catalyseurs ont été testés en transformation d'une charge C₅-C₆ dont la composition est la suivante (exprimée en % poids) :

| | |
|---|---|
| Paraffines | C₅ 90 % |
| | C₆ 5,4 % |
| Naphtènes | C₅ 3,7 % |
| | C₆ 0,9 % |

Les conditions opératoires sont les suivantes :

| | |
|---|---|
| - température | 470°C |
| - pression | 2,5 bars |
| - pph | 2,5 h-1 |

Les résultats du test sont reportés dans le tableau 1.

**Tableau 1**

| | | Sélectivités (% poids) | | | | | |
|---|---|---|---|---|---|---|---|
| Catalyseur | Conversion (% poids) | CH₄ | C₂H₆ + C₂H₄ | C₃H₈ + C₃H₆ | butanes + butènes | oléfines C₅+C₆ | Aromatiques |
| Catalyseur A (comparatif) | 55 | 4 | 11 | 19 | 17 | 39 | 10 |
| Catalyseur B (comparatif) | 90 | 29 | 25 | 15 | 19 | 0 | 12 |
| Catalyseur D | 85 | 8 | 14 | 17 | 12 | 1 | 48 |
| Catalyseur E (comparatif) | 79 | 9 | 10 | 18 | 15 | 5 | 43 |

On constate donc que le catalyseur D selon l'invention conduit à des conversions et à des sélectivités en produits aromatiques nettement supérieures à celles des catalyseurs comparatifs, notamment du catalyseur E.

### Exemple 5 : performances des catalyseurs sur charge en C₃

Les quatre catalyseurs A, B, D et E dont la préparation a été décrite précédemment, ont été testés en transformation du propane dans les conditions suivantes :

| | |
|---|---|
| - température | 465°C |
| - pression | atmosphérique |
| - pph | 1,0 h-1 |

Les résultats du test sont reportés dans le tableau 2.

**Tableau 2**

| | | Sélectivités (% poids) | | | | |
|---|---|---|---|---|---|---|
| Catalyseur | Conversion (% moles) | méthane | éthane + éthylène | propylène | butanes + butènes | Aroma tiques |
| Catalyseur A (comparatif) | 11 | 2 | 5 | 92 | 0 | 1 |
| Catalyseur B (comparatif) | 18 | 47 | 26 | 23 | 2 | 2 |
| Catalyseur D | 34 | 8 | 25 | 23 | 5 | 39 |
| Catalyseur E (comparatif) | 25 | 9 | 24 | 33 | 2 | 32 |

On constate donc que le catalyseur D selon l'invention conduit à des conversions et à des sélectivités en produits aromatiques nettement supérieures à celles des catalyseurs comparatifs, notamment du catalyseur E.

## Revendications

1. Catalyseur composite contenant :
- de 1 à 99% en poids (par rapport à la matrice du catalyseur) d'une zéolithe de structure MFI sous forme hydrogène, contenant dans sa charpente le silicium et au moins un élément choisi dans le groupe formé par l'aluminium et le gallium,
- une matrice,
- déposés sur la matrice au moins un métal noble de la famille du platine, et au moins un métal additionnel choisi dans le groupe constitué par l'étain, le germanium, et le plomb,
- déposé sur la matrice au moins un halogène choisi dans le groupe constitué par le fluor, le chlore, le brome et l'iode.

2. Catalyseur selon la revendication 1 et contenant en outre au moins un élément choisi dans le groupe constitué par les métaux alcalins et les métaux alcalino-terreux.

3. Catalyseur selon l'une des revendications 1 et 2 contenant en outre au moins un élément dopeur choisi dans le groupe constitué par le gallium et le zinc.

4. Catalyseur selon l'une des revendications 1 à 3 dans lequel ledit catalyseur contient, en poids par rapport à la matrice :
- de 0,01 à 2 % d'au moins un métal noble de la famille du platine,
- de 0,005 à 2 % d'étain dans le cas où le catalyseur contient de l'étain, ou de 0,005 à 0,7 % d'au moins un métal additionnel autre que l'étain.
- de 0,1 à 15 % d'au moins un halogène choisi dans le groupe constitué par le fluor, le chlore, le brome et l'iode.
- de 1 à 99% en poids d'une zéolithe de structure MFI sous forme hydrogène.

5. Catalyseur selon la revendication 4 renfermant au moins deux métaux additionnels, la concentration en métaux additionnels étant comprise entre 0,02 et 1,20 % en poids par rapport à la matrice.

6. Catalyseur selon l'une des revendications 1 à 5 et qui contient en outre, en poids par rapport à la matrice, de 0,01 à 4 % d'au moins un métal choisi dans le groupe constitué par les alcalins et les alcalino-terreux.

7. Catalyseur selon l'une des revendications 1 à 5 dont la zéolithe renferme au moins un élément dopeur choisi parmi le gallium ou le zinc, la teneur en gallium ou zinc étant comprise entre 0,01 et 10 % en poids par rapport à la zéolithe.

8. Utilisation du catalyseur composite défini selon l'une des revendications 1 à 7 pour l'aromatisation d'hydrocarbures contenant de 2 à 12 atomes de carbone par molécule.

## Claims

1. A composite catalyst containing:
- 1% to 99% by weight (with respect to the catalyst matrix) of a MFI structure zeolite in its hydrogen form, with a framework containing silicon and at least one element selected from the group formed by aluminium and gallium,
- a matrix,
- at least one precious metal from the platinum group and at least one additional metal selected from the group constituted by tin, germanium and lead, deposited on the matrix,
- at least one halogen deposited on the matrix and selected from the group constituted by fluorine, chlorine, bromine and iodine.

2. A catalyst according to claim 1, further containing at least one element selected from the group constituted by alkali metals and alkaline-earth metals.

3. A catalyst according to claim 1 or claim 2, further containing at least one doping element selected from the group constituted by gallium and zinc.

4. A catalyst according to any one or claims 1 to 3, wherein said catalyst contains, by weight with respect to the matrix:
- 0.01% to 2% of at least one precious metal from the platinum group,
- 0.005% to 2% of tin when the catalyst contains tin, or 0.005% to 0.7% of at least one additional metal other than tin,
- 0.1% to 15% of at least one halogen selected from the group constituted by fluorine, chlorine, bromine and iodine,
- 1% to 99% by weight of a MFI structure zeolite in its hydrogen form.

5. A catalyst according to claim 4, containing at least two additional metals, the concentration of additional metals being between 0,02% and 1.20% by weight with respect to the matrix.

6. A catalyst according to any one of claims 1 to 5, further containing, by weight with respect to the matrix, 0.01% to 4% of at least one metal selected from the group constituted by alkalis and alkaline-earths.

7. A catalyst according to any one of claims 1 to 5, wherein the zeolite contains at least one doping element selected from gallium or zinc, the concentration of gallium or zinc being between 0.01% and 10% by weight with respect to the zeolite.

8. The use of a composite catalyst as defined in any one of claims 1 to 7 for the aromatization of hydrocarbons containing 2 to 12 carbon atoms per molecule.

## Patentansprüche

1. Verbundkatalysator enthaltend:
von 1 - 99 Gew.-% (bezogen auf die Matrix des Katalysators) eines Zeoliths von MFI-Struktur in der Wasserstoff-Form, der in seinem Gerüst Silicium und wenigstens ein Element enthält, das aus der durch Aluminium und Gallium gebildeten Gruppe gewählt ist,
eine Matrix,
abgeschieden auf der Matrix wenigstens ein Edelmetall der Familie des Platins, wenigstens ein Zusatzmetall, das gewählt ist aus der Gruppe, die gebildet wird durch Zinn, Germanium, Blei,
abgeschieden auf der Matrix wenigstens ein Halogen, das gewählt ist aus der durch Fluor, Chlor, Brom und Jod gebildeten Gruppe.

2. Katalysator nach Anspruch 1, der außerdem wenigstens ein Element enthält, das gewählt ist aus der Gruppe, die durch die Alkalimetalle und die Erdalkalimetalle gebildet ist.

3. Katalysator nach einem der Ansprüche 1 und 2, der im übrigen wenigstens ein Dotierungselement enthält, das gewählt ist aus der durch Gallium und Zink gebildeten Gruppe.

4. Katalysator nach einem der Ansprüche 1 bis 3, bei dem dieser Katalysator in Gewicht, bezogen auf die Matrix, enthält:
von 0,01 bis 2 % wenigstens eines Edelmetalls der Familie des Platins,
von 0,005 bis 2 % Zinn für den Fall, wo der Katalysator Zinn enthält, oder 0,005 bis 0,7 % wenigstens eines Zusatzmetalls, das von Zinn verschieden ist,
von 0,1 bis 15 % wenigstens eines Halogens, das aus der durch Fluor, Chlor, Brom und Jod gebildeten Gruppe gewählt ist, und
von 1 bis 99 % eines Zeolithen von MFI-Struktur in der Wasserstoff-Form.

5. Katalysator nach Anspruch 4, der wenigstens zwei Zusatzmetalle umfaßt, wobei die Konzentration an Zusatzmetallen zwischen 0,02 bis 1,20 Gew.-%, bezogen auf die Matrix, liegt.

6. Katalysator nach einem der Ansprüche 1 bis 5, der im übrigen, in Gewicht bezogen auf die Matrix, von 0,01 bis 4 % wenigstens eines Metalls enthält, das aus der durch die Alkalimetalle und die Erdalkalimetalle gebildeten Gruppe gewählt ist.

7. Katalysator nach einem der Ansprüche 1 bis 5, dessen Zeolith wenigstens ein Dotierungselement umfaßt, das gewählt ist aus Gallium oder Zink, wobei der Gehalt an Gallium oder Zink zwischen 0,01 und 10 Gew.-%, bezogen auf den Zeolithen, beträgt.

8. Verwendung des gemäß einem der Ansprüche 1 bis 7 definierten Katalysators zur Aromatisierung von 2 bis 12 Kohlenstoffatome pro Molekül enthaltenden Kohlenwasserstoffen.
